(19) Europäisches Patentamt
European Patent Office
Office européen des brevets

(11) Veröffentlichungsnummer : **0 009 707**
**B1**

(12) **EUROPÄISCHE PATENTSCHRIFT**

(45) Veröffentlichungstag der Patentschrift : **23.12.81**

(21) Anmeldenummer : **79103486.1**

(22) Anmeldetag : **17.09.79**

(51) Int. Cl.³ : **C 07 D233/60**, C 07 D249/08, A 01 N 43/50, A 01 N 43/64// C07C49/163

(54) **Halogenierte 1-Azolyl-1-fluorphenoxy-butan-Derivate, Verfahren zu ihrer Herstellung, sie enthaltende Fungizide Mittel sowie ihre Verwendung als Fungizide.**

(30) Priorität : **28.09.78 DE 2842137**

(43) Veröffentlichungstag der Anmeldung : **16.04.80 (Patentblatt 80/08)**

(45) Bekanntmachung des Hinweises auf die Patenterteilung : **23.12.81 Patentblatt 81/51**

(84) Benannte Vertragsstaaten : **AT BE CH DE FR GB IT LU NL SE**

(56) Entgegenhaltungen :
**DE - A - 2 632 601**
**DE - A - 2 632 602**
**DE - A - 2 632 603**
**DE - A - 2 455 955**

(73) Patentinhaber : **BAYER AG**
***Zentralbereich Patente, Marken und Lizenzen***
**D-5090 Leverkusen 1, Bayerwerk (DE)**

(72) Erfinder : **Krämer, Wolfgang, Dr.**
**Am Eckbusch 39/162**
***D-5600 Wuppertal 1 (DE)***
Erfinder : **Büchel, Karl-Heinz, Dr.**
**Bergerheide 62**
**D-5600 Wuppertal 1 (DE)**
Erfinder : **Frohberger, Paul-Ernst, Dr.**
**Willi-Baumeister-Strasse 5**
**D-5090 Leverkusen (DE)**

**EP 0 009 707 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

## Halogenierte 1-Azolyl-1-fluorphenoxy-butan-Derivate, Verfahren zu ihrer Herstellung, Sie enthaltende Fungizide Mittel sowie ihre Verwendung als Fungizide

Die vorliegende Erfindung betrifft neue 1-Azolyl-1-fluorphenoxy-butan-Derivate, ein Verfahren zu ihrer Herstellung und ihre Verwendung als Fungizide.

Es ist bereits bekannt geworden, daß 1-Azolyl-1-chlorphenoxy-butan-Derivate, wie z.B. 4-Chlor-1-(4-chlorphenoxy)-3,3-dimethyl-1-(1,2,4-triazol-1-yl)- bzw. -(imidazol-1-yl)-butan-2-on und 4-Brom-1-(4-chlorphenoxy)-3,3-dimethyl-1-(imidazol-1-yl)-butan-2-ol gute fungizide Eigenschaften aufweisen (vergleiche DT-OS 26 32 602 (Le A 17 274) und DT-OS 26 32 603 (Le A 17 273)). Deren gute Wirkung ist jedoch, insbesondere bei niedrigen Aufwandmengen und -konzentrationen, nicht immer ganz befriedigend.

Es wurden neue halogenierte 1-Azolyl-1-fluorphenoxy-butan-Derivate der allgemeinen Formel

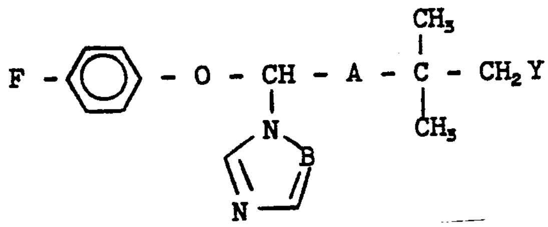

(I)

in welcher

A für eine Keto-Gruppe oder eine CH(OH)-Gruppe steht,

B für ein Stickstoffatom oder eine CH-Gruppe steht, und

Y für Chlor oder Brom steht,

und deren physiologisch verträglichen Salze gefunden. Sie weisen starke fungizide Eigenschaften auf.

Diejenigen Verbindungen der Formel (I), in welcher A für die CH(OH)-Gruppe steht, besitzen zwei asymmetrische Kohlenstoffatome ; sie können deshalb in den beiden geometrischen Isomeren (erythro- und threo-Form) vorliegen, die in unterschiedlichen Mengenverhältnissen anfallen können. In beiden Fällen liegen sie als optische Isomere vor. Sämtliche Isomeren werden erfindungsgemäß beansprucht.

Man erhält die halogenierten 1-Azolyl-1-fluorphenoxy-butan-Derivate der Formel (I), indem man Brometherketone der Formel

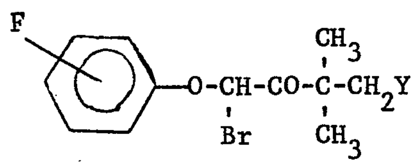

(II)

in welcher

Y die oben angegebene Bedeutung hat,

mit Azolen der Formel

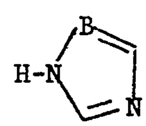

(III)

in welcher

B die oben angegebene Bedeutung hat,

in Gegenwart eines Säurebindemittels und in Gegenwart eines Verdünnungsmittels umsetzt, und gegebenenfalls die dabei erhaltenen Keton-Derivate in an sich bekannter Weise mit komplexen Borhydriden, gegebenenfalls in Gegenwart eines Verdünnungsmittels, reduziert.

Weiterhin können die erfindungsgemäß erhältlichen halogenierten 1-Azolyl-1-fluorphenoxy-butan-Derivate durch Umsetzen mit Säuren in die Salze überführt werden.

Ueberraschenderweise zeigen die erfindungsgemäßen Wirkstoffe eine erheblich höhere fungizide Wirksamkeit, insbesondere gegen Mehltauarten, als die aus dem Stand der Technik bekannten 1-Azolyl-1-chlorphenoxy-butan-Derivate, wie 4-Chlor-1-(4-chlorphenoxy)-3,3-dimethyl-1-(1,2,4-triazol-1-yl)- bzw. -(imidazol-1-yl)-butan-2-on und 4-Brom-1-(4-chlorphenoxy)-3,3-dimethyl-1-(imidazol-1-yl)-butan-2-ol, welches chemisch und wirkungsmäßig die naheliegendsten Verbindungen sind. Die erfindungsgemäßen Wirkstoffe stellen somit eine Bereicherung der Technik dar.

Verwendet man 1-Brom-4-chlor-3,3-dimethyl-1-(4-fluor-phenoxy)-butan-2-on und 1,2,4-Triazol als Ausgangsstoffe, so kann der Reaktionsablauf durch das folgende Formelschema wiedergegeben werden :

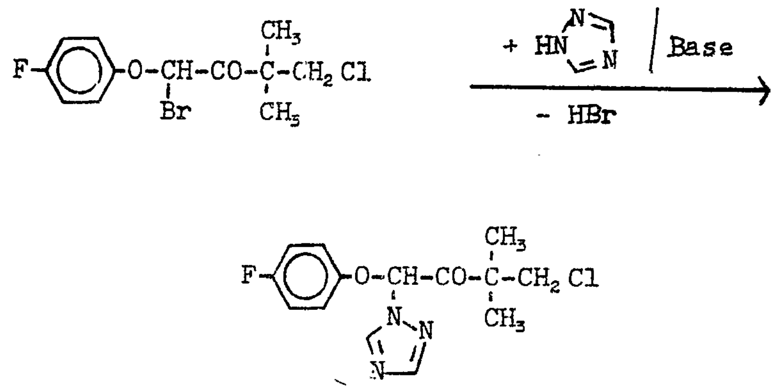


Verwendet man 4-Chlor-3,3-dimethyl-1-(4-fluorphenoxy)-1-(1,2,4-triazol-1-yl)-butan-2-on und Natriumborhydrid als Ausgangsstoffe, so kann der Reaktionsablauf durch das folgende Formelschema wiedergegeben werden :

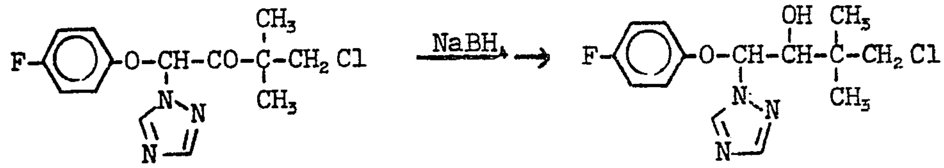


Die als Ausgangsstoffe zu verwendenden Brometherketone sind durch die Formel (II) allgemein definiert. In dieser Formel steht *Y* vorzugsweise für Chlor und Brom.

Die Brometherketone der Formel (II) sind noch nicht bekannt, können jedoch nach bekannten Verfahren erhalten werden, indem man z.B. Fluorphenol mit einem Bromketon der Formel

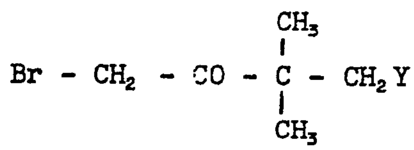
(IV)

in welcher

Y die oben angegebene Bedeutung hat,

umsetzt. Das noch verbliebene aktive Wasserstoffatom wird anschließend in üblicher Weise gegen Brom ausgetauscht (vergleiche auch die Herstellungsbeispiele).

Als Beispiele für die Ausgangsstoffe der Formel (II) seien genannt :

1-Brom-4-chlor-(4-fluorphenoxy)-3,3-dimethyl-butan-2-on 1,4-Dibrom-(4-fluorphenoxy)-3,3-dimethyl-butan-2-on als Salze für die Verbindungen der Formel (I) kommen Salze mit physiologisch verträglichen Säuren infrage. Hierzu gehörem vorzugsweise die Halogenwasserstoffsäure, wie z.B. die Chlorwasserstoffsäure und die Bromwasserstoffsäure, insbesondere die Chlorwasserstoffsäure, Phosphorsäure, Salpetersäure, mono- und bifunktionelle Carbonsäuren und Hydroxycarbonsäuren, wie z.B. Essigsäure, Zitronensäure, Sorbinsäure, Milchsäure, 1,5-Naphthalin-disulfonsäure.

Für die erfindungsgemäße Umsetzung kommen als Verdünnungsmittel vorzugsweise inerte organische Lösungsmittel infrage. Hierzu gehören vorzugsweise Ketone, wie Diäthylketon, insbesondere Aceton und Methyläthylketon ; Nitrile, wie Propionitril, insbesondere Acetonitril ; Alkohole, wie Aethanol oder Isopropanol ; Aether, wie Tetrahydrofuran oder Dioxan ; aromatische Kohlenwasserstoffe, wie Toluol und 1,3-Dichlorbenzol, Benzol ; Formamide, wie insbesondere Dimethylformamid ; und halogenierte Kohlenwasserstoffe, wie Methylenchlorid, Tetrachlorkohlenstoff oder Chloroform.

Die Umsetzung wird in Gegenwart eines Säurebinders vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise Triäthylamin, N,N-Dimethylcyclohexylamin, Dicyclohexylmethylamin, N,N-Dimethylbenzylamin, weiterhin Pyridin und Diazabicyclooctan. Vorzugsweise verwendet man einen entsprechenden Überschuß an dem Azol der Formel (III).

Die Reaktionstemperaturen können in einem größeren Bereich variiert werden. Im allgemeinen arbeitet man zwischen etwa 0 bis etwa 150 °C, vorzugsweise bei 60 bis 120 °C.

Bei der Durchführung des erfindungsgemäßen Verfahrens setzt man auf 1 Mol der Verbindungen der Formel (II) vorzugsweise 1 bis 2 Mol Azol und 1 bis 2 Mol Säurebinder ein. Zur Isolierung der Verbindungen der Formel (I) wird das Lösungsmittel abdestilliert, der Rückstand mit einem organischen Solvens aufgenommen und mit Wasser gewaschen. Die organische Phase wird über Natriumsulfat getrocknet und im Vakuum vom Lösungsmittel befreit. Der Rückstand wird durch Destillation bzw.

Umkristallisation gereinigt.

Für die erfindungsgemäße Reduktion kommen als Verdünnungsmittel für die erfindungsgemäße Umsetzung polare organische Lösungsmittel infrage. Hierzu gehören vorzugsweise Alkohole, wie Methanol, Aethanol, Butanol, Isopropanol, und Aether, wie Diäthyläther oder Tetrahydrofuran. Die Reaktion wird im allgemeinen bei 0 bis 30 °C, vorzugsweise bei 0 bis 20 °C durchgeführt. Hierzu setzt man auf 1 Mol der entsprechenden Ketoverbindung etwa 1 Mol eines Borhydrids, wie Natriumborhydrid oder Lithiumborhydrid, ein. Zur Isolierung der Verbindungen der Formel (I) wird der Rückstand z.B. in verdünnter Salzsäure aufgenommen, anschließend alkalisch gestellt und mit einem organischen Lösungsmittel extrahiert, oder nur mit Wasser versetzt und mit einem organischen Solvens ausgeschüttelt. Die weitere Aufarbeitung erfolgt in üblicher Weise.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Erysiphe-Arten eingesetzt werden. Hervorzuheben ist, daß die erfindungsgemäßen Wirkstoffe nicht nur eine protektive Wirkung entfalten, sondern auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid ; als feste Trägerstoffe kommen in Frage : z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z.B. nichtionogene und anionische Emulgatoren, wie Polyoxyäthylen-Fettsäure-Ester, Polyxyäthylen-Fettalkohol-Äther, z.B. Alkylarylpolyglykol-äther, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen,

Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,000 01 bis 0,1 Gew.-%, vorzugsweise von 0,000 1 bis 0,02 %, am Wirkungsort erforderlich.

### Beispiel A

Sproßbehandlungs-Test/Getreidemehltau/protektiv (blattzerstörende Mykose).

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator (Alkyl-aryl-polyglykol-ether) auf und gibt 975 Gewichtsteile Wasser hinzu. Das Konzentrat verdünnt man mit Wasser auf die gewünschte Endkonzentration der spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var. hordei.

Nach 6 Tagen Verweilzeit der Pflanzen bei einer Temperatur von 21-22 °C und einer Luftfeuchtigkeit von 80-90 % wertet man den Besatz der Pflanzen mit Mehltaupusteln aus. Der Befallsgrad wird in Prozent des Befalls der unbehandelten Kontrollpflanzen ausgedrückt. Dabei bedeutet 0 % keinen Befall und 100 % den gleichen Befallsgrad wie bei der unbehandelten Kontrolle. Der Wirkstoff ist umso wirksamer, je geringer der Mehltaubefall ist.

Wirkstoffe, Wirkstoffkonzentrationen in der Spritzbrühe und Befallsgrade gehen aus der nachfolgenden Tabelle hervor.

### Tabelle A

Sproßbehandlungs-Test/Getreidemehltau/protektiv

| Wirkstoffe | Wirkstoffkonzentration in der Spritzbrühe in Gew.% | Befall in % der unbehandelten Kontrolle |
|---|---|---|
| 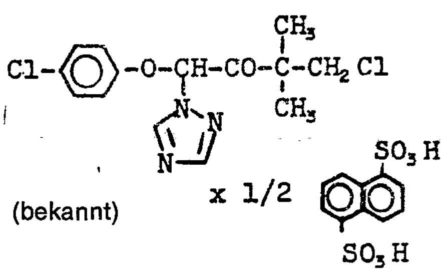 (bekannt) | 0,000 25 | 100 |
| 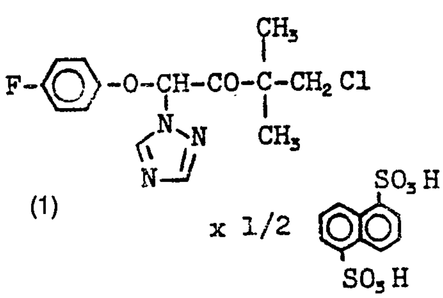 (1) | 0,000 25 | 37,5 |

Tabelle A (Fortsetzung)

Sproßbehandlungs-Test/Getreidemehltau/protektiv

| Wirkstoffe | Wirkstoffkonzentration in der Spritzbrühe in Gew.% | Befall in % der unbehandelten Kontrolle |
|---|---|---|
| 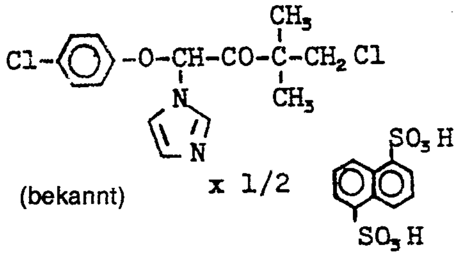 x 1/2 (bekannt) | 0,000 25 | 50,0 |
| 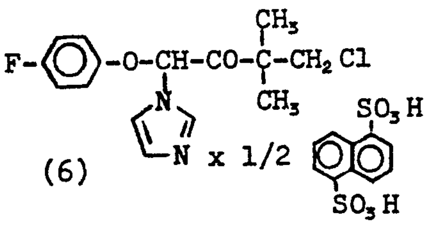 (6) x 1/2 | 0,000 25 | 10,0 |

Beispiel B

Gerstenmehltau-Test (Erysiphe graminis var. hordei)/systemisch (pilzliche Getreidesproßkrankheit).

Die Anwendung der Wirkstoffe erfolgt als pulverförmige Saatgutbehandlungsmittel. Sie werden hergestellt durch Abstrecken des jeweiligen Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3 × 12 Korn in Blumentöpfe 2 cm tief in ein Gemisch aus einem Volumteil Fruhstorfer Einheitserde und einem Volumteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var. hordei bestäubt und bei 21-22 °C und 80-90 % rel. Luftfeuchte und 16-stündiger Belichtung weiter kultiviert. Innerhalb von 6 Tagen bilden sich an den Blättern die typischen Mehltaupusteln aus.

Der Befallsgrad wird in Prozenten des Befalls der unbehandelten Kontrollpflanzen ausgedrückt. So bedeutet 0 % keinen Befall und 100 % den gleichen Befallsgrad wie bei der unbehandelten Kontrolle. Der Wirkstoff ist um so wirksamer je geringer der Mehltaubefall ist.

Wirkstoffe, Wirkstoffkonzentrationen im Saatgutbehandlungsmittel sowie dessen Aufwandmenge und der prozentuale Mehltaubefall gehen aus der nachfolgenden Tabelle hervor.

Tabelle B

| Gerstenmehltau-Test Wirkstoffe | (Erysiphe graminis var. hordei)/systemisch Wirkstoffkonzentration im Beizmittel in Gew.-% | Beizmittelaufwandmenge in g/kg Saatgut | Befall in % der unbehandelten Kontrolle |
|---|---|---|---|
| 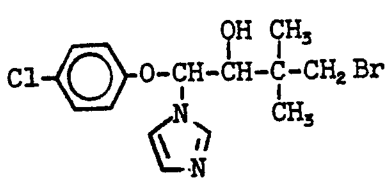 (bekannt) | 25 | 10 | 50,0 |

Tabelle B

Gerstenmehltau-Test (Erysiphe graminis var. hordei)/systemisch

| Wirkstoffe | Wirkstoffkonzentration im Beizmittel in Gew.-% | Beizmittelaufwandmenge in g/kg Saatgut | Befall in % der unbehandelten Kontrolle |
|---|---|---|---|
| 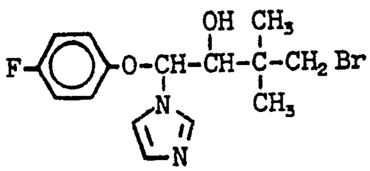 (7) | 25 | 10 | 12,5 |

## Herstellungsbeispiele

### Beispiel 1

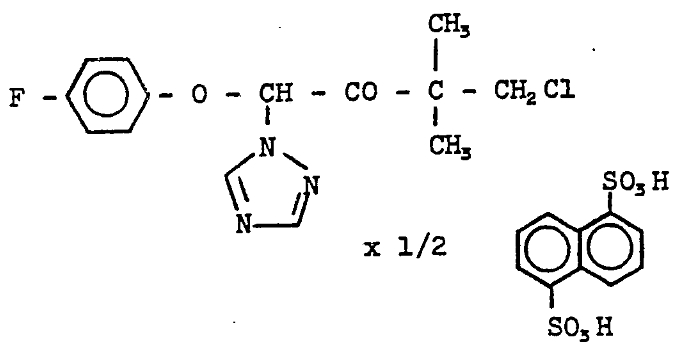

x 1/2

57 g (0,177 Mol) 1-Brom-4-chlor-3,3-dimethyl-1-(4-fluorphenoxy)-butan-2-on werden in 600 ml absolutem Acetonitril gelöst, 24,2 g (0,35 Mol) 1,2,4-Triazol zugegeben und 6 Stunden unter Rückfluß erhitzt. Das Lösungsmittel wird im Wasserstrahlvakuum abdestilliert, der Rückstand in 700 ml Methylenchlorid aufgenommen, die organische Phase zweimal mit 1,5 l Wasser extrahiert und über Natriumsulfat getrocknet. Das Lösungsmittel wird im Wasserstrahlvakuum abdestilliert und das zurückbleibende Oel in 200 ml Aceton aufgenommen. Man gibt 18 g (0,1 Mol) 1,5-Naphthalindisulfonsäure, gelöst in 100 ml Aceton, zu und saugt den entstandenen Niederschlag ab. Man erhält 47 g (55,7 % der Theorie) 4-Chlor-3,3-dimethyl-1-(4-fluorphenoxy)-1-(1,2,4-triazol-1-yl)-butan-2-on-naphthalindisulfonat-(1,5) vom Schmelzpunkt 200 °C.

### Herstellung des Ausgangsproduktes

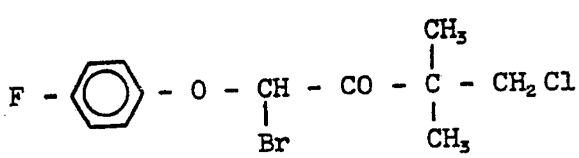

100,2 g (0,617 Mol) 1,4-Dichlor-3,3-dimethyl-butan-2-on werden bei Siedetemperatur zu einer Lösung von 56 g (0,5 Mol) p-Fluorphenol und 85 g Kaliumcarbonat in 700 ml Aceton getropft. Man läßt 6 Stunden unter Rückfluß rühren, saugt ab und destilliert das Lösungsmittel ab. Man erhält 101,5 g (83 % der Theorie) 4-Chlor-3,3-dimethyl-1-(4-fluorphenoxy)-butan-2-on, das roh in 600 ml Methylenchlorid gelöst wird. Dann werden bei Raumtemperatur 66,3 g (0,415 Mol) Brom so zugetropft, daß Entfärbung eintritt. Man rührt 30 Minuten nach, destilliert das Lösungsmittel im Wasserstrahlvakuum ab und gibt 100 ml Pentan zu, wobei der Rückstand kristallisiert. Man erhält 114 g (85 % der Theorie) 1-Brom-4-chlor-3,3-dimethyl-1-(4-fluorphenoxy)-butan-2-on vom Schmelzpunkt 74 °C.

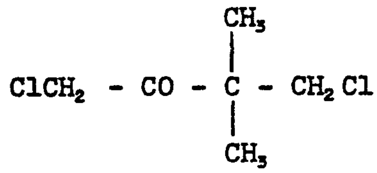

1 050 g (7,8 Mol) 78 %-iges 4-Chlor-3,3-dimethyl-butan-2-on werden in 6 l Methylenchlorid gelöst und bei 10 bis 11 °C während 6 Stunden 450 g Chlor eingeleitet. Danach wird 30 Minuten nachgerührt, mit Stickstoff der überschüssige Chlorwasserstoff ausgetrieben und das Reaktionsgemisch über eine 60 cm Füllkörperkolonne destilliert. Man erhält 832 g (63 % der Theorie) 1,4-Dichlor-3,3-dimethyl-butan-2-on vom Siedepunkt 50-54 °C/0,1 Torr mit einer 75 %-igen Reinheit.

Beispiel 2

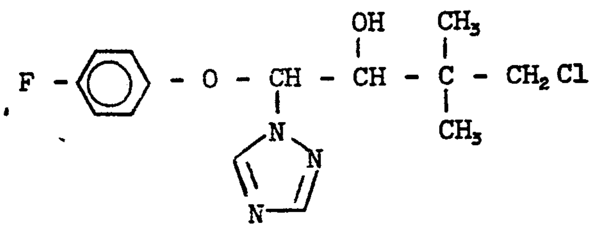

Zu 25 g (0,053 Mol) 4-Chlor-3,3-dimethyl-1-(4-fluor-phenoxy)-1-(1,2,4-triazol-1-yl)-butan-2-on-naphthalindisulfonat (Beispiel 1), gelöst in 400 ml Methanol, werden portionsweise 4,75 g (0,12 Mol) Natriumborhydrid gegeben und 15 Stunden bei Raumtemperatur nachgerührt. Unter Kühlung werden 15 ml konzentrierte Salzsäure vorsichtig eingetropft, 2 Stunden bei Raumtemperatur gerührt und das Lösungsmittel zur Hälfte im Wasserstrahlvakuum abdestilliert. Das Reaktionsgemisch wird auf 400 ml gesättigte Natriumhydrogencarbonatlösung gegeben, mit 300 ml Methylenchlorid extrahiert, die organische Phase abgetrennt, mit zweimal je 100 ml Wasser gewaschen, über Natriumsulfat getrocknet und durch Abdestillieren des Lösungsmittels eingeengt. Der Rückstand kristallisiert nach Zugabe von 100 ml Diisopropylether. Man erhält 9,1 g (36,4 % der Theorie) 4-Chlor-3,3-dimethyl-1-(4-fluorphenoxy)-1-(1,2,4-triazol-1-yl)-butan-2-ol vom Schmelzpunkt 87-90 °C.

Analog werden die nachfolgenden Verbindungen der Formel

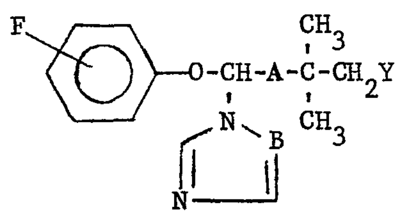

erhalten :

| Bsp. Nr | A | B | Y | Schmelzpunkt(°C) |
|---|---|---|---|---|
| 3 | CO | N | Br | 180 ($\times\frac{1}{2}$NDS) |
| 4 | CHOH | N | Br | 100 |
| 5 | CO | CH | Br | 241 ($\times\frac{1}{2}$NDS) |
| 6 | CO | CH | Cl | 254 ($\times\frac{1}{2}$NDS) |
| 7 | CHOH | CH | Br | 116 |
| 8 | CHOH | CH | Cl | 115 |

NDS = 1,5-Naphthalindisulfonsäure

**Ansprüche**

1. 1-Azolyl-1-fluorphenoxy-butan-Derivate der allgemeinen Formel

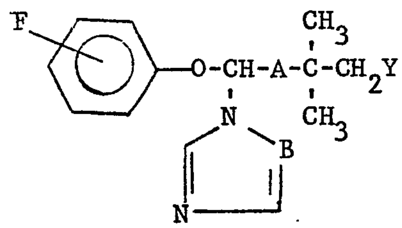

(I)

in welcher
A für eine Ketogruppe oder eine CH(OH)-Gruppe steht,
B für ein Stickstoffatom oder eine CH-Gruppe steht, und
Y für Chlor oder Brom steht,
und deren physiologisch verträglichen Salze.

2. Verfahren zur Herstellung von 1-Azolyl-1-fluorphenoxy-butan-Derivaten der allgemeinen Formel

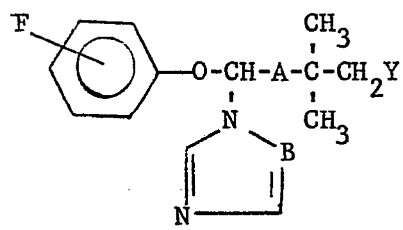

(I)

in welcher
A für eine Ketogruppe oder eine CH(OH)-Gruppe steht,
B für ein Stickstoffatom oder eine CH-Gruppe steht, und
Y für Chlor oder Brom steht,
dadurch gekennzeichnet, daß man Brometherketone der Formel

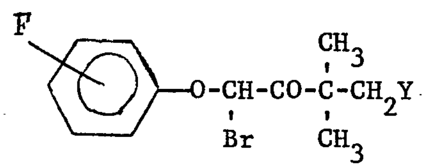

(II)

in welcher
Y die oben angegebene Bedeutung hat,
mit Azolen der Formel

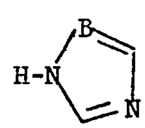

(III)

in welcher
B die oben angegebene Bedeutung hat,
in Gegenwart eines Säurebindemittels und in Gegenwart eines Verdünnungsmittels umsetzt, und gegebenenfalls die dabei erhaltenen Keton-Derivate in an sich bekannter Weise mit komplexen Borhydriden, gegebenenfalls in Gegenwart eines Verdünnungsmittels, reduziert.

3. Fungizide Mittel, gekennzeichnet durch einen Gehalt an mindestens einem 1-Azolyl-1-fluorphenoxy-butan Derivat gemäß Anspruch 1.

4. Verwendung von 1-Azolyl-1-fluorphenoxy-butan-Derivaten gemäß Anspruch 1 zur Bekämpfung von Pilzen.

**Claims**

1. 1-Azolyl-1-fluorophenoxy-butane derivatives of the general formula

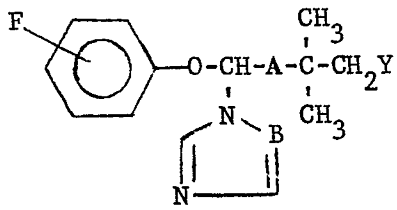

(I)

in which

A represents a keto group or a CH(OH) group,

B represents a nitrogen atom or a CH group and

Y represents chlorine or bromine,

and their physiologically acceptable salts.

2. Process for the preparation of 1-azolyl-1-fluorophenoxy-butane derivatives of the general formula

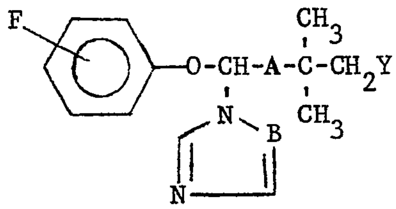

(I)

in which

A represents a keto group or a CH(OH) group,

B represents a nitrogen atom or a CH group and

Y represents chlorine or bromine,

characterised in that bromoether-ketones of the formula

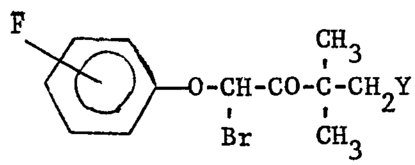

(II)

in which

Y has the meaning indicated above,

are reacted with azoles of the formula

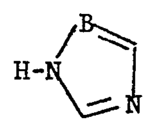

(III)

in which

B has the meaning indicated above,

in the presence of an acid-binding agent and in the presence of a diluent, and the ketone derivatives thereby obtained are optionally reduced with complex borohydrides in a manner which is in itself known, if appropriate in the presence of a diluent.

3. Fungicidal agents, characterised in that they contain at least one 1-azolyl-1-fluorophenoxy-butane derivative according to Claim 1.

4. Use of 1-azolyl-1-fluorophenoxy-butane derivatives according to Claim 1 for combating fungi.

**Revendications**

1. Dérivés de 1-azolyl-1-fluorophénoxy-butane de formule

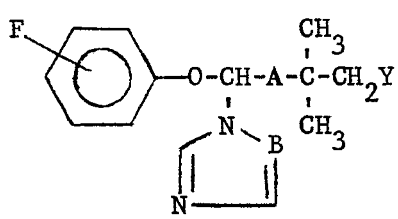

(I)

dans laquelle

A représente un groupe céto ou un groupe CH(OH),

B représente un atome d'azote ou un groupe CH et

Y représente le chlore ou le brome,

et leurs sels physiologiquement acceptables.

2. Procédé pour la préparation de dérivés de 1-azolyl-1-fluorophénoxy-butane de formule générale

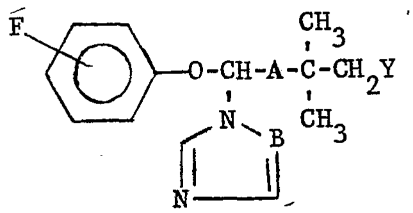

(I)

dans laquelle

A représente un groupe céto ou un groupe CH(OH),

B représente un atome d'azote ou un groupe CH et

Y représente le chlore ou le brome,

caractérisé en ce que l'on fait réagir des bromoéthercétones de formule

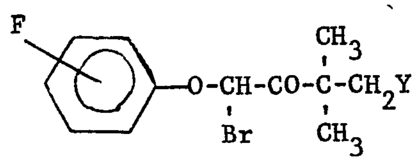

(II)

dans laquelle

Y a la signification indiquée ci-dessus, avec des azoles de formule

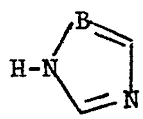

(III)

dans laquelle

B a la signification indiquée ci-dessus, en présence d'un accepteur d'acide et en présence d'un agent diluant, et en réduisant éventuellement, de manière connue, les dérivés de cétones ainsi obtenus avec des borohydrures complexes, éventuellement en présence d'un agent diluant.

3. Agents fongicides, caractérisés en ce qu'ils contiennent au moins un dérivé de 1-azolyl-1-fluorophénoxy-butane selon la revendication 1.

4. Utilisation de dérivés de 1-azolyl-1-fluorophénoxy-butane selon la revendication 1, pour la lutte contre les champignons.